Europäisches Patentamt

European Patent Office

Office européen des brevets

(11) Numéro de publication: **0 474 539 B1**

# (12) FASCICULE DE BREVET EUROPEEN

(45) Date de publication de fascicule du brevet: **11.01.95**

(51) Int. Cl.6: **C07C 15/00**, C07C 5/41, C07C 2/00

(21) Numéro de dépôt: **91402310.6**

(22) Date de dépôt: **23.08.91**

Le dossier contient des informations techniques présentées postérieurement au dépôt de la demande et ne figurant pas dans le présent fascicule.

(54) **Procédé d'aromatisation des hydrocarbures contenant de 5 a 9 atomes de carbone par molécule en présence d'un catalyseur particulier.**

(30) Priorité: **03.09.90 FR 9010947**

(43) Date de publication de la demande:
**11.03.92 Bulletin 92/11**

(45) Mention de la délivrance du brevet:
**11.01.95 Bulletin 95/02**

(84) Etats contractants désignés:
**DE GB IT NL**

(56) Documents cités:
**EP-A- 0 351 311**
**WO-A-89/04818**
**FR-A- 2 144 702**
**US-A- 4 435 283**

(73) Titulaire: **INSTITUT FRANCAIS DU PETROLE**
**4, Avenue de Bois Préau**
**F-92502 Rueil-Malmaison (FR)**

(72) Inventeur: **Bournonville, Jean-Paul**
**1,Ouartier Puch-Long-Rousson**
**F-30340 Salindres (FR)**
Inventeur: **Raatz, Francis**
**25, rue de la Carrière**
**F-57500 Saint Avold (FR)**
Inventeur: **Juguin, Bernard**
**46, avenue du Stade**
**F-92500 Rueil Malmaison (FR)**

## Description

La présente invention concerne un procédé d'aromatisation des hydrocarbures comprenant entre 5 et 9 atomes de carbone par molécule en présence d'un catalyseur composite comprenant une zéolithe de structure MFI contenant du silicium, de l'aluminium et au moins un métal de la famille du platine auquel sont ajoutés au moins un métal additionnel choisi dans le groupe constitué par l'étain, le germanium, l'indium et le plomb. Une matrice amorphe peut être ajoutée à ce catalyseur en vue notamment de la mise en forme.

Les catalyseurs à base de zéolithes dopés au gallium, zinc, platine, sont connus pour être actifs et sélectifs en aromatisation du propane et du butane. Classiquement les hydrocarbures à plus de 6 atomes de carbone par molécule sont transformés en aromatiques par reformage catalytique en utilisant des catalyseurs du type alumine acide dopée au platine, métal auquel on peut ajouter de l'étain, du rhénium etc. Ces catalyseurs de réformage sont cependant très peu performants pour l'aromatisation des hydrocarbures comprenant 5 atomes de carbone par molécule, et dans une moindre mesure pour ceux comprenant 6 atomes de carbone par molécule. Il y a donc un grand intérêt pratique à trouver des catalyseurs performants pour l'aromatisation de coupes riches en hydrocarbures du type $C_5$-$C_6$.

La réaction d'aromatisation des hydrocarbures comprenant plus de 5 atomes de carbone par molécule en présence de catalyseurs zéolithiques a déjà fait l'objet de plusieurs brevets et publications. Plusieurs systèmes catalytiques à base de zéolithe MFI sont revendiqués, ces systèmes peuvent être distingués par les ajouts qu'ils contiennent. Schématiquement on peut distinguer :

i) les systèmes contenant du gallium (G. Berti, J. Moore, L. Salusinszki, D. Seddon, Aust. J. Chem., 42, p. 2095, 1989).

ii) les systèmes contenant du zinc (0. Anunziata, 0. Orio, L. Pierella, M. Aguirre, React. kin. Catal. Lett., 39(1), 75, 1989 ; J. Kanai, N. Kawata, J. Catal., 114, 284, 1988).

Ces systèmes souffrent tous d'un défaut important : à savoir une sélectivité élevée en méthane. Pour améliorer les performances de ces systèmes catalytiques plusieurs solutions ont été proposées : ajout de platine (Z. Jin, Y. Makino, A. Miyamoto, T. Inui, Chem. Express, 2, p. 515, 1987). L'utilisation d'une zéolithe MFI non acide dopée avec divers éléments métalliques a également été revendiquée (Y. Chen, et al. WO 8904818).

Par ailleurs, dans la demande de brevet européen EP - A-458674 , la demanderesse revendique un catalyseur comprenant, d'une part, une zéolithe MFI, et, d'autre part, un support ou une matrice généralement amorphe sur lequel est déposé un métal noble de la famille du platine, et, au moins, un métal additionnel choisi dans le groupe constitué par l'étain, le germanium, l'indium et le plomb, ledit support contenant également au moins un métal alcalin ou au moins un métal alcalino-terreux choisi dans le groupe constitué par le lithium, le sodium, le potassium, le rubidium, le césium, le baryum, le calcium, le béryllium, le magnésium et le strontium ; ce catalyseur est utilisé pour l'aromatisation des hydrocarbures comprenant 2 à 4 atomes de carbone par molécule.

On a découvert que la fixation du métal noble de la famille du platine (métal hydrogénant) et du métal additionnel choisi dans le groupe constitué par l'étain, le germanium, l'indium et le plomb, directement sur la zéolithe, par des techniques telles que l'imprégnation, l'échange ou tout autre technique connue de l'homme de l'art, conduisait à une amélioration des performances catalytiques en aromatisation des hydrocarbures comprenant 5 à 9 atomes de carbone par molécule.

La zéolithe de structure MFI dudit catalyseur (qui est acide) employé dans la présente invention, peut être préparée par toutes les techniques connues dans l'art antérieur. La synthèse de ladite zéolithe MFI peut être réalisée en milieu classique $OH^-$, en présence ou non de structurants organiques et/ou d'alcool. La synthèse de la zéolithe MFI en milieu $OH^-$ selon les techniques connues dans l'art antérieur est largement décrite dans le document suivant : Synthesis of High Silica Zeolites, P. Jacobs and J. Martens, Studies in Surface Science and Catalysis, volume 33, Elsevier Editor, 1987. La zéolithe MFI peut également être synthétisée dans des milieux moins classiques comme par exemple le milieu fluorure (Brevet Européen EP-A-172068 de C.F.R.).

Après synthèse, la zéolithe MFI est transformée en une forme hydrogène par élimination totale ou partielle des composés organiques et/ou des cations alcalins ou alcalino-terreux qu'elle contient éventuellement après la synthèse. Toutes les techniques connues dans l'art antérieur peuvent être utilisées pour passer à la forme hydrogène, comme par exemple les calcinations sous atmosphère oxydante ou non, les échanges ioniques suivis ou non de calcination, les traitements chimiques divers etc.

Toutes les zéolithes MFI synthétisées dans le système Si-Al conviennent pour la présente invention. Cependant leur rapport atomique Si/Al sera généralement supérieur à 7, de préférence supérieur à 25 et de préférence compris entre 40 et 1 000.

Le métal hydrogénant est ensuite déposé sur la zéolithe MFI. N'importe quel métal du groupe VIII de la classification périodique des éléments peut convenir, mais le platine est le métal préféré.

Le platine peut être introduit de différentes façons, par exemple :
- sous forme de complexe tétrammine par échange cationique ;
- sous forme d'acide hexachloroplatinique par imprégnation.

Le platine (ou éventuellement un autre métal noble du groupe du platine) peut être ainsi incorporé dans la zéolithe par imprégnation de cette zéolithe à l'aide d'une solution adéquate aqueuse ou non renfermant un sel ou un composé du métal noble. Le platine est généralement introduit dans la zéolithe sous forme d'acide chloroplatinique, mais peuvent également être utilisés des composés tels que le chloroplatinate d'ammonium, le dichlorure de platine dicarbonyle, l'acide hexahydroxyplatinique, le chlorure de palladium, le nitrate de palladium.

Parmi les composés du ou des métaux de la famille du platine que l'on emploie dans la présente invention, on peut citer également à titre d'exemple les complexes ammoniés.

On citera en particulier dans le cas du platine : les sels de platine IV hexamine de formule $(Pt(NH_3)_6)X_4$ où X est un atome d'halogène choisi dans le groupe formé par le fluor, le chlore, le brome et l'iode et de préférence X est un atome de chlore ; les sels de platine IV halogénopentammines de formule $(PtX(NH_3)_5)$-$X_3$ ; les sels de platine IV tétrahalogénodiammines de formule $Pt X_4(NH_3)_2$ où X a la signification donnée ci-dessus ; les complexes du platine avec les halogènes-polycétones et les composés halogénés polycétoniques de formule $H(Pt(aca)_2X)$ dans lesquels X a la signification donnée ci-dessus et aca représente le reste de formule $C_5H_7O_2$ dérivé de l'acétylacétone.

L'introduction du métal noble de la famille du platine est de préférence effectuée par imprégnation à l'aide d'une solution aqueuse ou organique de l'un des composés organométalliques cités ci-dessus. Parmi les solvants organiques utilisables on peut citer les hydrocarbures paraffiniques, naphténiques ou aromatiques et les composés organiques halogénés ayant par exemple de 1 à 12 atomes de carbone dans leur molécule. On citera en particulier le n-heptane, le méthylcyclohexane, le toluène et le chloroforme. On peut aussi utiliser des mélanges de ces solvants.

L'élément (ou métal additionnel M) choisi dans le groupe constitué de l'étain, du germanium, du plomb et de l'indium peut être introduit par l'intermédiaire de composés tels que par exemple les chlorures, les bromures et le nitrate d'étain, les halogénures, nitrate, acétate et carbonate de plomb, le chlorure et l'oxalate de germanium, le nitrate et le chlorure d'indium.

Le métal additonnel M peut être introduit avant ou après l'introduction du métal noble. S'il est introduit avant le métal noble, le composé utilisé sera choisi dans le groupe constitué par les halogénures, nitrates, acétates, carbonates, oxalates du métal additionnel. L'introduction sera avantageusement effectuée en solution aqueuse. Dans ce cas, avant de procéder à l'introduction du métal noble, on procède à une calcination sous air à une température comprise entre 400 et 1000 degrés centigrades.

Le métal additionnel M peut être introduit après l'introduction du métal noble sous la forme d'au moins un composé organique choisi dans le groupe formé par les complexes, en particulier les complexes polycétoniques, des métaux M et les hydrocarbylmétaux tels que les alkyles, les cycloalkyles, les aryles, les alkylaryles et les arylalkyles métaux.

L'introduction du métal M est avantageusement effectuée à l'aide d'une solution dans un solvant organique du composé organométallique dudit métal M. On peut également employer des composés organohalogénés des métaux M. Comme composés de métaux M on citera en particulier le tétrabutyl-étain, le tétraméthyl-étain, le tétrapropyl-germanium, le tétraéthyl-plomb, l'acétylacétonate d'indium, le triphénylindium.

Le solvant d'imprégnation est choisi dans le groupe constitué par les hydrocarbures paraffiniques, naphténiques ou aromatiques contenant de 6 à 12 atomes de carbone par molécule et les composés organiques halogénés contenant de 1 à 12 atomes de carbone par molécule. On peut citer le n-heptane, le méthylcyclohexane, le toluène et le chloroforme. On peut utiliser des mélanges des solvants définis ci-dessus.

Cette méthode d'introduction du métal M a déjà été décrite dans le brevet US-A-4548918. Mais la combinaison de la méthode d'introduction du métal de la famille du platine et de la méthode d'introduction du métal M engendre une synergie particulière.

La zéolithe MFI du catalyseur employé dans l'invention renferme en poids (a) 0,01 à 2 % et plus particulièrement 0,1 à 0,5 % d'au moins un métal noble de la famille du platine, (b) 0,005 à 0,60 %, et de préférence 0,01 à 0,50 % d'étain et/ou 0,005 à 0,70 % de préférence 0,01 à 0,6 % et plus particulièrement 0,02 à 0,50 %, d'au moins un métal choisi dans le groupe constitué par le germanium, le plomb et l'indium.

Lorsqu'il y a au moins deux métaux de la famille étain, germanium, plomb et indium, la teneur globale en métaux de cette famille est de 0,02 à 1,20 % et de préférence 0,02 à 1,0 % et plus particulièrement de

EP 0 474 539 B1

0,02 à 0,8 %.

On peut utiliser soit une solution commune des métaux que l'on désire déposer sur la zéolithe, soit des solutions distinctes pour le métal de la famille du platine et pour le ou les métaux additionnels. Quand on utilise plusieurs solutions, on peut procéder à des séchages et/ou calcinations intermédiaires. On termine habituellement par une calcination par exemple entre environ 500 et 1000°C, de préférence en présence d'oxygène libre, par exemple en effectuant un balayage d'air.

A l'issue de la préparation du catalyseur, celui-ci est généralement calciné entre 450 et 1000°C mais le catalyseur, à l'issue de la calcination peut subir avantageusement un traitement d'activation sous hydrogène à haute température, par exemple 300-500°C, afin d'obtenir une phase métallique plus active. La procédure de ce traitement sous hydrogène consiste par exemple en une montée lente de la température sous courant d'hydrogène jusqu'à la température maximale de réduction, comprise par exemple entre 300 et 500°C et de préférence entre 350 et 450°C, suivie d'un maintien pendant 1 à 6 heures à cette température.

Ce type de préparation du catalyseur conduit à un solide dans lequel les métaux sont répartis de façon homogène dans tout le volume du grain de catalyseur, et sont dans un état métallique après le traitement de réduction sous balayage d'hydrogène entre 300 et 500°C et maintien pendant 1 à 6 heures sous hydrogène à la température finale choisie.

Une méthode avantageuse de préparation des catalyseurs peut être utilisée selon les étapes suivantes :

(a) On imprègne la zéolithe MFI avec une solution aqueuse d'un composé d'un métal choisi dans le groupe constitué par l'étain, le germanium, l'indium et le plomb ;
(b) On sèche le produit obtenu à l'étape (a) ;
(c) On calcine le produit obtenu à l'étape (b) ;
(d) On imprègne le produit obtenu à l'étape (c) par une solution d'acétylacétonate de platine dans le toluène ;
(e) On sèche le produit obtenu à l'étape (d) ;
(f) On calcine le produit obtenu à l'étape (e) ;
(g) On réduit sous courant d'hydrogène le produit obtenu à l'étape (f).

Une autre méthode avantageuse de préparation des catalyseurs peut être réalisée selon les étapes suivantes :

(a) On imprègne la zéolithe MFI avec une solution aqueuse d'un composé d'un métal choisi dans le groupe constitué par l'étain, l'indium, le germanium et le plomb ;
(b) On sèche le produit obtenu à l'étape (a) ;
(c) On calcine le produit obtenu à l'étape (b) ;
(d) On imprègne avec une solution ammoniacale de chlorure de platine tétrammine le produit obtenu à l'étape (c) ;
(e) On sèche le produit obtenu à l'étape (d) ;
(f) On calcine le produit obtenu à l'étape (e) ;
(g) On réduit le produit obtenu à l'étape (f) sous un courant d'hydrogène.

Une autre méthode avantageuse de préparation des catalyseurs peut être réalisée selon les étapes suivantes :

(a) On imprègne la zéolithe MFI avec une solution ammoniacale de chlorure de platine tétrammine ;
(b) On sèche le produit obtenu à l'étape (a) ;
(c) On calcine le produit obtenu à l'étape (b) ;
(d) On réduit sous courant d'hydrogène le produit obtenu à l'étape (c) ;
(e) On met en contact le produit obtenu à l'étape (d) avec un solvant hydrocarboné et avec ledit composé organique dudit métal M, par exemple en immergeant la masse dans un solvant hydrocarboné renfermant déjà le composé organique ou en immergeant la masse dans un solvant hydrocarboné et en injectant ensuite dans le mélange obtenu une solution du composé organique dudit métal M dans un solvant hydrocarboné et par exemple celui dans lequel ladite masse a été immergée ;
(f) On réduit sous courant d'hydrogène le produit obtenu à l'étape (e).

Le catalyseur peut également contenir un support ou une matrice amorphe, choisi, par exemple, parmi les oxydes de magnésium, d'aluminium, de titane, de zirconium, de thorium, de silicium, de bore, pris seuls ou en mélange entre eux. On peut aussi utiliser du charbon.

Le support préféré est l'alumine ; la surface spécifique de l'alumine peut avantageusement être comprise entre 50 et 600 $m^2$/g, de préférence entre 150 et 400 $m^2$/g.

La zéolithe contenant les divers métaux précédemment décrits peut être mise en forme avec le support par toute technique connue de tout homme du métier : pastillage, extrusion, dragéification, coagulation en goutte, séchage par atomisation par exemple.

4

Le catalyseur contient alors 1 à 99 % en poids de zéolithe contenant les différents métaux, le complément à 100 % étant constitué par le support ou la matrice amorphe.

Le catalyseur est utilisé dans un procédé d'aromatisation d'hydrocarbures renfermant 5 à 9 atomes de carbone par molécule, dans les conditions opératoires habituelles d'un tel procédé.

Les exemples suivants illustrent l'invention sans en limiter la portée.

Exemple 1

On se propose de transformer une charge constituée d'un mélange d'hydrocarbures contenant 5 ou 6 atomes de carbone en présence d'un catalyseur à base d'une zéolithe MFI de rapport atomique Si/Al égal à 45 contenant du platine et un métal choisi dans le groupe constitué par l'étain, le germanium, l'indium et le plomb.

. Préparation de la zéolithe MFI :

La zéolithe MFI est synthétisée en présence de structurant organique en utilisant une des recettes connues dans l'art antérieur (USA-A-3.702.886). Cette zéolithe est transformée en une forme H par les traitements suivants :
- calcination sous un mélange air-azote (10 % d'oxygène dans le mélange) à 550°C pendant 4 heures,
- trois échanges dans $NH_4NO_3$ 5N à 100°C,
- calcination sous air à 530°C pendant 5 heures sous un débit de 5 l/h/g.

Le rapport atomique Si/Al de la zéolithe HMFI est égal à 45 et son volume poreux mesuré par adsorption d'azote à 77 K est supérieur à 0,160 $cm^3$/g.

Les métaux sont déposés sur la zéolithe MFI selon les procédures suivantes :

. Catalyseur A

(a) on imprègne la zéolithe MFI par une solution aqueuse de chlorure d'étain de façon à ce que la concentration finale du catalyseur en étain soit égale à 0,25 % poids.
(b) On sèche le produit obtenu à l'étape (a) pendant 1 heure à 100-120°C.
(c) On calcine le produit obtenu à l'étape (b) pendant 2 heures à 530°C.
(d) On imprègne le produit obtenu à l'étape (c) par une solution aqueuse d'acide hexachloroplatinique de façon à obtenir 0,3 % de platine sur le catalyseur final.
(e) On sèche une heure à 100-120°C le produit obtenu.
(f) On calcine 2 heures à 530°C.
Catalyseur A : 0,3 % Pt et 0,25 % Sn/MFI.

. Catalyseur B

On imprègne la zéolithe MFI selon la même procédure que pour le catalyseur (A) si ce n'est que l'on remplace le chlorure d'étain par l'acétate d'étain. La suite de la procédure demeure inchangée.
Catalyseur B : 0,3 % Pt et 0,25 % Sn/MFI.

. Catalyseur C

On imprègne la zéolithe MFI selon la même procédure que pour le catalyseur B si ce n'est que le platine est imprégné au moyen d'une solution ammoniacale de chlorure de platine tétrammine. Le reste de la procédure demeure inchangé.
Catalyseur C : Pt : 0,3 % et Sn : 0,25 %/MFI.

. Catalyseur D (catalyseur comparatif)

On imprègne la zéolithe MFI par une solution ammoniacale de chlorure de platine tétrammine, de façon à obtenir une concentration en platine égale à 0,3 % poids sur le catalyseur final.
Catalyseur D : Pt : 0,3 %/MFI.

Exemple 2

On a soumis les catalyseurs préparés ci-dessus à un test d'aromatisation d'une coupe $C_5$-$C_6$ de composition suivante (% poids) :

$$\text{Paraffines} \qquad \begin{cases} C_5 & : \quad 90,0 \ \% \\ C_6 & : \quad 5,4 \ \% \end{cases}$$

$$\text{Naphtènes} \qquad \begin{cases} C_5 & : \quad 3,7 \ \% \\ C_6 & : \quad 0,9 \ \% \end{cases}$$

Les conditions opératoires sont les suivantes :

| - Température | 480 ° C |
|---|---|
| - Pression | 0,25 Mégapascal |
| - pph | 3 h$^{-1}$ |

Les résultats des tests comparatifs des catalyseurs A à D et de la zéolithe MFI seule sont reportés dans le tableau 1.

Tableau 1

| Catalyseur | Conversion % poids | Sélectivité (% poids) | | | | |
|---|---|---|---|---|---|---|
| | | $CH_4$ | $C_2H_6$ + $C_2H_4$ | $C_3H_8$ + $C_3H_6$ | $C_4H_{10}$ + $C_4H_8$ | Aromatiques |
| Zéolithe MFI | 92 | 35 | 25 | 15 | 20 | 5 |
| Catalyseur A | 90 | 5 | 12 | 15 | 7 | 61 |
| Catalyseur B | 92 | 5 | 13 | 14 | 5 | 63 |
| Catalyseur C | 94 | 4 | 10 | 12 | 8 | 66 |
| Catalyseur D | 95 | 25 | 20 | 10 | 15 | 30 |

Exemple 3

Pour préparer des catalyseurs E, F et G respectivement, on suit une procédure d'imprégnation strictement identique à celle retenue pour la préparation du catalyseur C si ce n'est que l'on remplace l'acétate d'étain par l'oxalate de germanium (catalyseur E), le nitrate de plomb (catalyseur F) et le nitrate d'indium (catalyseur G), le reste de la procédure étant inchangé.

- Catalyseur E : Pt = 0,3 % et Ge : 0,20 %/MFI
- Catalyseur F : Pt = 0,3 % et Pb : 0,35 %/MFI
- Catalyseur G : Pt = 0,3 % et In : 0,25 %/MFI

Dans le tableau 2 sont repris l'ensemble des résultats des tests d'aromatisation de la coupe $C_5$-$C_6$ réalisés avec ces catalyseurs dans les mêmes conditions que celles définies dans l'exemple 2.

Tableau 2

| Catalyseur | Conversion % poids | Sélectivité (% poids) | | | | |
|---|---|---|---|---|---|---|
| | | $CH_4$ | $C_2H_6$ + $C_2H_4$ | $C_3H_8$ + $C_3H_6$ | $C_4H_{10}$ + $C_4H_8$ | Aroma-tiques |
| Catalyseur D | 95 | 25 | 20 | 10 | 15 | 30 |
| Catalyseur E | 90 | 5 | 13 | 14 | 4 | 64 |
| Catalyseur F | 91 | 5 | 11 | 16 | 5 | 63 |
| Catalyseur G | 93 | 5 | 12 | 15 | 6 | 62 |
| Catalyseur C | 94 | 4 | 10 | 12 | 8 | 66 |

Exemple 4

Le catalyseur D préparé ci-dessus est réduit sous courant d'hydrogène pendant deux heures à 450°C ; on immerge 100 grammes de ce catalyseur dans 300 cm$^3$ de n-heptane. Ensuite on injecte, dans le n-heptane contenant le catalyseur, 2 grammes d'une solution de tétra n-butyl étain dans le n-heptane (à 10 % en étain). Le contact entre le catalyseur au platine et la solution de tétra n-butyl étain est maintenu pendant 6 heures à la température de reflux de l'heptane. La solution d'imprégnation est ensuite évacuée et l'on procède à 3 lavages par du n-heptane pur à la température de reflux du n-heptane. Le catalyseur est alors séché. Il peut ensuite subir soit une calcination sous air pendant 2 heures à 500°C suivie d'une réduction sous courant d'hydrogène à 450°C pendant deux heures avant d'être chargé dans le réacteur, soit subir une réduction directe sous courant d'hydrogène à 450°C pendant 2 heures avant d'être chargé dans le réacteur.

On obtient ainsi le catalyseur H :

Catalyseur H : Pt : 0,3 % et Sn : 0,2 %/MFI

Les résultats d'aromatisation de la coupe $C_5/C_6$, dans des conditions identiques à celles de l'exemple 2 sont rassemblés dans le tableau 3.

Tableau 3

| Catalyseur | Conversion % poids | Sélectivité (% poids) | | | | |
|---|---|---|---|---|---|---|
| | | $CH_4$ | $C_2H_6$ + $C_2H_4$ | $C_3H_8$ + $C_3H_6$ | $C_4H_{10}$ + $C_4H_8$ | Aromatiques |
| Catalyseur D | 95 | 25 | 20 | 10 | 15 | 30 |
| Catalyseur C | 94 | 4 | 10 | 12 | 8 | 66 |
| Catalyseur H | 95 | 4 | 8 | 10 | 8 | 70 |

Ainsi, une bonne sélectivité en produits aromatiques est obtenue avec les catalyseurs conformes à ceux employés dans la présente invention (A, B, C, E, F, G et H).

**Revendications**

1. Procédé d'aromatisation d'hydrocarbures renfermant 5 à 9 atomes de carbone par molécule caractérisé en ce qu'il est effectué en présence d'au moins un catalyseur renfermant une zéolithe de structure MFI, acide et de rapport atomique Si/Al compris entre 40 et 1 000, contenant au moins un métal noble de la famille du platine et au moins un métal additionnel choisi dans le groupe constitué par l'étain, le germanium, le plomb et l'indium.

2. Procédé selon la revendication 1 dans lequel ladite zéolithe contient au moins un métal noble de la famille du platine en une quantité de 0,01 % à 2 % poids et au moins un métal additionnel choisi dans le groupe constitué par l'étain, le germanium, le plomb et l'indium en une quantité de 0,005 à 0,60 % poids pour l'étain et en une quantité de 0,005 à 0,70 % poids pour le germanium, le plomb ou l'indium.

3. Procédé selon l'une des revendications 1 et 2 dans lequel ladite zéolithe contient au moins un métal noble de la famille du platine en une quantité de 0,1 à 0,5 % poids et au moins un métal additionnel choisi dans le groupe constitué par l'étain, le germanium, le plomb et l'indium en une quantité de 0,01 à 0,5 % poids pour l'étain et en une quantité de 0,01 à 0,60 % poids pour le germanium, le plomb ou l'indium.

4. Procédé selon l'une des revendications 1 à 3 dans lequel ladite zéolithe contient à titre de métaux additionnels de l'étain et au moins un métal choisi dans le groupe constitué par le germanium, le plomb et l'indium, la teneur totale en métaux additionnels de ladite zéolithe étant de 0,02 à 1,20 % poids.

5. Procédé selon l'une des revendications 1 à 4 dans lequel ledit catalyseur renferme en outre une matrice.

6. Procédé selon la revendication 5 dans lequel ledit catalyseur renferme en poids 1 à 99 % de ladite zéolithe et 99 à 1 % de ladite matrice.

7. Procédé selon l'une des revendications 5 et 6 dans lequel ladite matrice est l'alumine.

**Claims**

1. Process for the aromatization of hydrocarbons containing 5 to 9 carbon atoms per molecule, characterized in that it is carried out in the presence of at least one catalyst containing a MFI structure zeolite, acid and which Si/Al atomic ratio is between 40 and 1000, containing at least one noble metal from from the platinum family and at least one additional metal chosen from the group constituted by tin, germanium, lead and indium.

2. Process according to claim 1, wherein said zeolite contains at least one noble metal from the platinum family in a quantity of approximately 0.01 to 2% by weight and at least one additional metal chosen from within the group constituted by tin, germanium, lead and indium in a quantity of 0.005 to 0.60% by weight for the tin and a quantity of 0.005 to 0.70% by weight for the germanium, lead or indium.

3. Process according to one of the claims 1 and 2, wherein said zeolite contains at least one noble metal from the platinum family in a quantity of approximately 0.1 to 0.5% by weight and at least one additional metal chosen from within the group constituted by tin, germanium, lead and indium in a quantity of 0.01 to 0.50% by weight for the tin and in a quantity of 0.01 to 0.60% by weight for the germanium, lead or indium.

4. Process according to any one of the claims 1 to 3, wherein said zeolite contains as additional metals tin and at least one metal chosen from within the group constituted by germanium, lead and indium, the total additional metal content of said zeolite being 0.02 to 1.20% by weight.

5. Process according to one of the claims 1 to 4, wherein the catalyst also contains a matrix.

6. Process according to claim 5 containing by weight 1 to 99% of said zeolite and 99 to 1% of said matrix.

7. Process according to either of the claims 5 and 6, wherein said matrix is alumina.

**Patentansprüche**

1. Verfahren zur Aromatisierung von Kohlenwasserstoffen, welche 5 bis 9 Kohlenstoffe pro Molekül umfassen, dadurch gekennzeichnet, daß es in Gegenwart wenigstens eines Katalysators durchgeführt wird, der einen sauren Zeolith mit MFI-Struktur und einem Si/Al-Atomverhältnis, das zwischen 40 und 1000 liegt, wenigstens ein Edelmetall der Platinfamilie und wenigstens ein zusätzliches Metall enthält, das aus der Gruppe, bestehend aus Zinn, Germanium, Blei und Indium, ausgewählt ist.

2. Verfahren nach Anspruch 1, worin der Zeolith wenigstens ein Edelmatall der Platinfamilie in einer Menge von 0,01 bis 2 Gew.-% und wenigstens ein zusätzliches Metall, das aus der Gruppe, bestehend aus Zinn, Germanium, Blei und Indium, ausgewählt ist, in einer Menge von 0,005 bis 0,60 Gew.-% für Zinn und einer Menge von 0,005 bis 0,70 Gew.-% für Germanium, Blei oder Indium, enthält.

3. Verfahren nach Anspruch 1 oder 2, worin der Zeolith wenigstens ein Edelmatall der Platinfamilie in einer Menge von 0,1 bis 0,5 Gew.-% und wenigstens ein zusätzliches Metall, ausgewählt aus der Gruppe bestehend aus Zinn, Germanium, Blei und Indium, in einer Menge von 0,01 bis 0,5 Gew.-% für Zinn und einer Menge von 0,01 bis 0,60 Gew.-% für Germaium, Blei oder Indium enthält.

4. Verfahren nach einem der Ansprüche 1 bis 3, worin der Zeolith einen Gehalt an zusätzlichen Metallen von Zinn und wenigstens einem Metall, das aus der Gruppe, bestehend aus Germaium, Blei und Indium, ausgewählt ist, aufweist, wobei der Gesamtgehalt an zusätzlichen Metallen des Zeoliths 0,02 bis 1,20 Gew.-% beträgt.

5. Verfahren nach einem der Ansprüche 1 bis 4, worin der Katalysator des weiteren eine Matrix umfaßt.

6. Verfahren nach Anspruch 5, worin der Katalysator 1 bis 99 Gew.-% des Zeoliths und 99 bis 1 Gew.-% der Matrix umfaßt.

7. Verfahren nach einem der Ansprüche 5 bis 6, worin die Matrix Aluminiumoxid ist.